(19) Europäisches Patentamt
European Patent Office
Office européen des brevets

(11) Veröffentlichungsnummer: **0 005 196**
**A1**

(12) **EUROPÄISCHE PATENTANMELDUNG**

(21) Anmeldenummer: **79101121.6**

(22) Anmeldetag: **12.04.79**

(51) Int. Cl.²: **C 07 C 35/08,** A 61 K 7/46, C 07 C 47/32, C 07 C 43/18, C 07 C 49/30, C 07 C 69/14, C 07 C 69/24, C 11 D 3/16, C 11 D 3/20

(30) Priorität: **05.05.78 DE 2819594**

(43) Veröffentlichungstag der Anmeldung: **14.11.79 Patentblatt 79/23**

(84) Benannte Vertragsstaaten: **CH DE FR GB NL**

(71) Anmelder: **BASF Aktiengesellschaft, Carl-Bosch-Strasse 38, D-6700 Ludwigshafen (DE)**

(72) Erfinder: **Gnad, Josef, Dr., Londoner Ring 55, D-6700 Ludwigshafen (DE)**
Erfinder: **Hoffmann, Werner, Dr., Ringstrasse 11 c, D-6701 Neuhofen (DE)**
Erfinder: **Schaefer, Eberhard, Berliner Strasse 21 c, D-6700 Ludwigshafen (DE)**
Erfinder: **Schulze, Joachim, Dr., Sternstrasse 50, D-6700 Ludwigshafen (DE)**
Erfinder: **Schuster, Ludwig, Dr., Weinheimer Strasse 44, D-6703 Limburgerhof (DE)**

(54) **Octylcyclohexanderivate, ihre Herstellung und ihre Verwendung als Riechstoffe.**

(57) 4-Octyl-cyclohexanol, 4-Octyl-cyclohexanol-carbonsäureester, 4-Octyl-cyclohexanol-methyläther, 4-Octyl-cyclohexanon sowie 4-Octyl-cyclohexanal als neue Octyl-cyclohexanderivate.

4-Octyl-cyclohexanol erhält man in an sich bekannter Weise durch katalytische Hydrierung von 7-Octyl-phenol, seine Ester und den Methyläther durch Veresterung bzw. Methylierung.

Das erhaltene 4-Octyl-cyclohexanol läßt sich durch Dehydrieren in an sich bekannter Weise in das 4-Octyl-cyclohexanon überführen, aus welchem durch Umsetzen mit Halogenessigestern der Glycidester und aus diesem durch alkalische Hydrolyse und anschließende Säurespaltung das 4-Octyl-cyclohexanal hergestellt werden kann.

Die 4-Octyl-cyclohexanderivate können als Duftstoffe mit holzartigen Geruchsnoten in kosmetischen Zubereitungen aller Art sowie in Wasch- und Reinigungsmitteln oder als Duftverbesserer in Leimen, Anstrichsmitteln und ähnlichen technischen Erzeugnissen verwendet werden.

EP 0 005 196 A1

BASF Aktiengesellschaft O. Z. 0050/033160

# Octylcyclohexanderivate, ihre Herstellung und ihre Verwendung als Riechstoffe

Die vorliegende Erfindung betrifft neue Octylcyclohexanderivate der allgemeinen Formel I

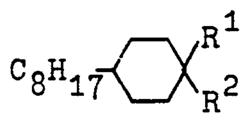

(I),

in der $R^1$ für H und
$R^2$ für -OH; -CHO; $-O-CO-CH_3$; $-O-CO-C_2H_5$; $-O-CO-C_3H_7$ oder $-O-CH_3$ steht

oder aber $R^1$ und $R^2$ zusammen für =O stehen und der Octylrest $C_8H_{17}$ im wesentlichen als 2,2,3,3-Tetramethylbutylrest vorliegt, sowie Verfahren zu deren Herstellung und deren Verwendung als Duftstoff mit holzartigen Geruchsnoten in kosmetischen Zubereitungen aller Art sowie in Wasch- und Reinigungsmittel oder als Duftverbesserer in Leimen, Anstrichmitteln und ähnlichen technischen Erzeugnissen.

Bekannte ähnliche Riechstoffe sind p-tert.-Butylcyclohexylacetat, p-tert.-Butyl-cyclohexanon und p-tert.-Butylcyclohexanol, deren Markvolumen bei ca. 500 Jahrestonnen liegt. Die neuen Verbindungen sind geruchlich

Rr/ro

etwas weniger intensiv als die bekannten Verbindungen, dafür jedoch wesentlich länger haftend und anhaltend. Die Duftrichtung der neuen Octylcyclohexanderivate ist ähnlich der der tert.-Butylcyclohexanderivate, jedoch unterscheiden sie sich in den Nuancen. Besonders interessant ist das Octylcyclohexanon, das überraschenderweise einen sehr angenehmen Duft nach Holz besitzt.

Es wurde gefunden, daß man die neuen Octylcyclohexanderivate technisch sehr vorteilhaft herstellen kann, indem man das durch Friedel-Crafts-Reaktion aus Phenol und Diisobutylen technisch sehr gut zugängliche Octylphenol in an sich bekannter Weise katalytisch hydriert und das erhaltene Octylcyclohexanol in an sich bekannter Weise in seine Ester, Äther bzw. ins Octylcyclohexanon oder den Octylcyclohexanylaldehyd überführt. Entsprechend der Herstellung durch Friedel-Crafts-Reaktion aus Phenol und Diisobutylen liegt der Octylrest im Octylphenol und somit der Octylrest in allen Verbindungen der Formel I im wesentlichen, in der 2,2,3,3-Tetramethylbutylform vor, was durch $^{1}H$-NMR und $^{13}C$-NMR-Spektroskopie nachgewiesen werden kann.

Gegenstand der Anmeldung ist demnach auch ein Verfahren zur Herstellung des Octylcyclohexanderivates der allgemeinen Formel I gemäß Anspruch 1, in der $R^1$ für H und $R^2$ für -OH steht, das dadurch gekennzeichnet ist, daß man das entsprechende Octylphenol in an sich bekannter Weise katalytisch hydriert;

ein Verfahren zur Herstellung des Octylcyclohexanderivates der allgemeinen Formel I gemäß Anspruch 1, in der $R^1$ für H und $R^2$ für $-O-CO-CH_3$, $-O-CO-C_2H_5$ oder $-O-CO-C_3H_7$ steht, das dadurch gekennzeichnet ist, daß man

A das entsprechende Octylphenol in an sich bekannter Weise katalytisch hydriert und

B das erhaltene Octylcyclohexanol in an sich bekannter Weise verestert;

ein Verfahren zur Herstellung des Octylcyclohexanderivates der allgemeinen Formel I gemäß Anspruch 1, in der $R^1$ für H und $R^2$ für $-O-CH_3$ steht, das dadurch gekennzeichnet ist, daß man

A das entsprechende Octylphenol in an sich bekannter Weise katalytisch hydriert und

C das erhaltene Octylcyclohexanol in an sich bekannter Weise methyliert;

ein Verfahren zur Herstellung des Octylcyclohexanderivates der allgemeinen Formel I gemäß Anspruch 1, in der $R^1$ und $R^2$ zusammen für =O stehen, das dadurch gekennzeichnet ist, daß man

A das entsprechende Octylphenol in an sich bekannter Weise katalytisch hydriert und

D das erhaltene Octylcyclohexanol in an sich bekannter Weise mit Chromschwefelsäure, Mangandioxid oder Nickelperoxid oxydiert oder aber katalytisch dehydriert;

sowie ein Verfahren zur Herstellung des Octylcyclohexanderivates der allgemeinen Formel I gemäß Anspruch 1, in der $R^1$ für H und $R^2$ für $-C\lessgtr^{O}_{H}$ steht, das dadurch gekennzeichnet ist, daß man

A das entsprechende Octylcyclohexanol in an sich bekannter Weise katalytisch hydriert,

D das erhaltene Octylcyclohexanol in an sich bekannter Weise zum Octylcyclohexanon dehydriert,

E aus dem Octylcyclohexanon durch Umsetzen mit Halogenessigestern in an sich bekannter Weise den Glycidester herstellt und

F diesen durch alkalische Hydrolyse und Säurespaltung zum Aldehyd abbaut.

Zu A Die Hydrierung des Octylphenols zum Octylcyclohexanol kann mit üblichen Hydrierungskatalysatoren durchgeführt werden. Genannt seien z.B. Katalysatoren, die eins oder mehrere der Metalle Pt, Pd, Rh, Ir, Ni, Co bzw. deren Oxide enthalten. Als Katalysatorträger können oberflächenaktive Substanzen verwendet werden, wie z.B. Bimsstein, Aluminiumoxid, Kieselgel, Aktivkohle, Zeolithe. Die Reaktion kann in Lösungsmitteln wie z.B. Butanol, Dioxan, Tetrahydrofuran durchgeführt werden.

Geeignete Hydriertemperaturen sind 50 bis 300°C, vorzugsweise 100 bis 250°C. Der Wasserstoffdruck kann zwischen 1 bis 400, insbesondere zwischen 150 und 300 bar liegen. Die Verweilzeit ist vom Druck und der Reaktionstemperatur abhängig; sie schwankt zwischen 0,5 und 50 Stunden, insbesondere zwischen 5 bis 20 Stunden.

Zu B Die Veresterung des Octylcyclohexanols kann auf übliche Weise mit Ameisensäure, Essigsäure bzw. Propionsäure erfolgen. Man kann jedoch auch übliche Acylierungsmittel wie Acetylchlorid, Propionylchlorid oder die entsprechenden Anhydride verwenden.

Bei der Veresterung des Octylcyclohexanols mit den Carbonsäuren setzt man dem Reaktionsgemisch im allgemeinen zwecks Erhöhung der Carbonylaktivität der Carbonsäuren geringe Mengen einer starken Säure wie $H_2SO_4$, HCl, Sulfonsäuren oder sauren Ionenaustauschern zu. Auch das Entfernen des bei der Reaktion gebildeten Wassers z.B. durch azeotropes Abdestillieren mit Toluol oder Cyclohexan ist von Vorteil. Die Carbonsäure verwendet man in Mengen von 1,1 bis 1,5 Mol pro Mol Alkohol.

Wesentlich leichter als mit den Carbonsäuren selbst erfolgt die Umsetzung mit Carbonsäurechloriden oder -anhydriden. Auch hierbei empfiehlt sich der Zusatz einer Säure oder einer Base als Katalysator oder bei Verwendung von Carbonsäurechloriden der Zusatz eines tertiären Amins, wie Triäthylamin zum Abfangen der gebildeten Chlorwasserstoffsäure.

Eine andere vorteilhafte Methode zur Veresterung des Octylcyclohexanols besteht in der säurekatalysierten Umesterung z.B. mit Essigsäuremethylester, bei der der gebildete niedere Alkohol, z.B. Methanol, ständig aus dem Reaktionsgemisch abdestilliert wird.

Bezüglich weiterer Einzelheiten über Veresterungsverfahren verweisen wir beispielsweise auf "Organikum", 14. Auflage, VEB Deutscher Verlag der Wissenschaften, Berlin 1975, S. 440-449.

Zu C Die Herstellung des Octylcyclohexanylmethyläthers erfolgt durch Umsetzen mit einem üblichen Alkylierungsmittel, wie Dimethylsulfat, Methyltosylat, insbesondere aber mit einem Methylhalogenid.

Die Umsetzung mit Alkylhalogeniden erfolgt nach der Art der bekannten Williamson'schen Äthersynthese, die beispielsweise in Houben-Weyl, "Methoden der organischen Chemie", 4. Auflage, Band 6/3, Seiten 26 ff. näher beschrieben ist.

Mit Vorteil geht man so vor, daß man das erhaltene Octylcyclohexanol zunächst mit überschüssigem Natriumhydrid, Natriumamid oder Lithiumbutyl in das Alkoholat überführt und zu dem erhaltenen Reaktionsgemisch das entsprechende Alkylierungsmittel zutropft.

Die Reaktion wird bei Temperaturen von 0 bis 50°C, vorzugsweise bei Temperaturen von 10 bis 30°C durchgeführt. Nach Zugabe des Methylhalogenids läßt man das Reaktionsgemisch im allgemeinen noch etwa 4 bis 6 Stunden bei Temperaturen von 10 bis 20°C unter Rühren nachreagieren.

Das Alkylierungsmittel verwendet man im allgemeinen in Mengen von etwa 1 bis 1,25 Mol pro Mol des zu alkylierenden Alkohols.

Zu D Die Dehydrierung des Octylcyclohexanols zum Octylcyclohexanon kann in an sich bekannter Weise sowohl katalytisch als auch durch chemische Oxidationsmittel erfolgen. Als geeignete Dehydrierungskatalysatoren seien Silber, Kupfer, Zink, Chrom, Nickel, Kobalt, Platin und Palladium, vorzugsweise Silber, Kupfer, Platin und Palladium, als solche oder in Verbindungen bzw. Legierungen genannt. Die Umsetzung wird im allgemeinen bei Temperaturen von 200 bis 400°C und vorzugsweise in der Gasphase durchgeführt.

Bezüglich weiterer Einzelheiten über die Herstellung von Ketonen durch katalytische Dehydrierung sekundärer Alkohole verweisen wir auf "Methoden der organischen Chemie", Houben-Weyl, Band 7/2a, Georg Thieme Verlag Stuttgart, Seiten 699-714.

Recht gut geeignet zur Herstellung des Octylcyclohexanons ist auch die Oxidation des Octylcyclohexanols mittels Chromschwefelsäure. Bei diesem Verfahren wird das Octylcyclohexanol zweckmäßig in einem unter den Reaktionsbedingungen inerten Lösungsmittel gelöst und diese Lösung unter Kühlung bei Temperaturen von 20 bis 60°C, vorzugsweise 30 bis 45°C mit einem Chrom-Schwefelsäure-Wassergemisch versetzt.

Bezüglich weiterer Einzelheiten über dieses Verfahren, insbesondere über die Zusammensetzung und Herstellung bewährter Chrom-Schwefelsäure-Wassergemische verweisen wir auf "Methoden der organischen Chemie", Houben--Weyl, Band 7/2a, Georg Thieme-Verlag, Stuttgart, Seiten 718 bis 739, insbesondere 722 bis 725.

Zu E Zur Überführung des Octylcyclohexanons in den Octylcyclohexylcarbonaldehyd setzt man dieses zunächst in Gegenwart eines Kondensationsmittels mit Chloressigsäuremethylester zu dem Glycidester der Formel

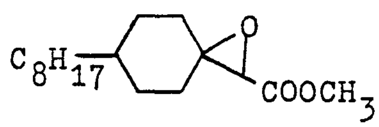

um. Die Glycidestersynthese wird zweckmäßig in Äther, Benzol oder Xylol durchgeführt. Als Kondensationsmittel haben sich Alkoholate, Natrium und vor allem Natriumamid und Kalium-tert.-butylat in tert.-Butylalkohol bewährt.

Zu F Die Überführung des Glycidesters in den Octylcyclohexylcarbonaldehyd wird in der Regel in zwei Stufen vorgenommen. Zunächst wird mit wäßriger oder alkoholischer Alkalilauge zunächst die Estergruppe verseift. Anschließend spaltet man die Glycidsäure durch Erhitzen mit verdünnter Salzsäure oder Schwefelsäure, oder man zersetzt thermisch unter stark vermindertem Druck, wobei ein Zusatz von Kupfer oder Kupferchromid vorteilhaft ist.

Bezüglich weiterer Einzelheiten über diese sogenannte Methode von Darzens-Erlenmeyer jr.-Claisen verweisen wir auf "Methoden der organischen Chemie", Houben-Weyl, Band 7/1 Georg Thieme-Verlag Stuttgart, Seite 326-329.

Die neuen Octylcyclohexanderivate der allgemeinen Formel I sind wertvolle Duftstoffe für kosmetische Zubereitungen aller Art sowie für Wasch- und Reinigungsmittel und wertvolle Duftverbesserer für Leime, Anstrichmittel und ähnliche technische Erzeugnisse. Besondere Bedeutung hat das Octylcyclohexanon mit seinem angenehmen Holzgeruch. Sie können technisch auf relativ einfache und damit billige Weise vollsynthetisch hergestellt werden.

Beispiel 1

a) In einen Rollautoklaven von 5 l Inhalt wurden 2220 g p-Octylphenol, 400 g Katalysator (25 bis 42 % Co-oxid + 11 % $Na_2CO_3$ auf Bimssilicat) sowie 132 g Kalziumoxid gegeben und anschließend in der Kälte 100 bar Wasserstoff aufgepreßt. Anschließend wurde auf 200°C erhitzt und der Wasserstoffdruck auf 200 bar ergänzt. Die Temperatur und der Druck (durch Nachpressen von Wasserstoff) wurden so lange konstant gehalten bis keine Wasserstoffaufnahme mehr erfolgte. Der Autoklav wurde während der Reaktion in Bewegung gehalten. Die Reaktionszeit lag bei etwa 25 Stunden. Der Autoklaveninhalt wurde anschließend über eine grobe Filternutsche gegeben, wobei der Katalysator und nicht verbrauchtes Kalziumoxid abgetrennt wurden. Das Reaktionsprodukt wurde durch Rektifikation gereinigt, wobei 1709 g Hauptlauf erhalten wurden (Ausbeute 75 % d.Th.); Kp = 116 bis 130°C/2 bis 3 mbar. Geruch: schwach holzig, leicht blumig.

b) In einem Autoklaven wurden 500 g p-Octylphenol in 100 ml Dioxan gelöst, mit 1,5 g Rutheniumoxidhydrat (50 % Ru) versetzt und bei 130°C und 300 bar Wasserstoffdruck unter gutem Rühren hydriert. Die Wasserstoffaufnahme war innerhalb von 5 Stunden beendet. Man entspannte den Autoklaven, ließ abkühlen und destillierte den Autoklaveninhalt. Es wurden 509 g Octylcyclohexanol erhalten (Ausbeute: 99 %). Kp: 110 - 115°C/0,1 mbar.

Beispiel 2

424 g (2 Mol) eines gemäß Beispiel 1 hergestellten Octylcyclohexanols wurden mit 224 g (2,2 Mol) Essigsäureanhydrid versetzt und bei einem auf 100 mbar verminderten Druck zum Sieden erhitzt. Die hierbei freigesetzte Essigsäure wurde gleichzeitig über eine 1 m Füllkörperkolonne abdestilliert. Nach Beendigung der Essigsäureabspaltung wurde der Druck im Reaktionsgefäß auf etwa 15 Torr gesenkt und das Reaktionsprodukt abdestilliert. Durch Rektifikation erhielt man 432 g reines Octylcyclohexylacetat vom Siedepunkt Kp = 153 bis 161°C/15 mbar; $n_D^{25}$ = 1,4641.
Geruch: balsamisch, holzig, mild blumig, feiner als der von tert.-Butylcyclohexylacetat.

Beispiel 3

504 g (2,38 Mol) eines gemäß Beispiel 1 hergestellten Octylcyclohexanols wurden in 500 ml Toluol gelöst und bei 35 bis 40°C unter kräftigem Rühren mit einer Chromschwefelsäure folgender Zusammensetzung versetzt: 1 475 g Wasser, 492 g konz. Schwefelsäure und 423 g (1,42 Mol) Natriumbichromat. Während des Zulaufs der Chromschwefelsäure mußte mit einem Eisbad gekühlt werden. Nach Beendigung des Zulaufs ließ man noch 2 Stunden bei 40°C rühren. Dann wurde die organische Schicht abgetrennt, die wäßrige Phase noch einmal mit 500 ml Toluol extrahiert und anschließend die vereinigten Toluollösungen mit Sodalösung und Wasser neutral gewaschen. Das nach Abdestillieren des

Toluols bei 50°C und 50 mbar verbleibende Produkt wird durch Rektifikation gereinigt. Man erhält 455 g Octylcyclohexanon (Ausbeute 91 %).
Kp 100 bis 103°C/0,1 mbar; $n_D^{25}$ = 1,4721
Geruch: herb holzig, pudrig, sandelholzartig

Nach 'HNMR- und $^{13}$C-NMR-Spektroskopie liegt das Produkt fast ausschließlich in folgender Form vor:

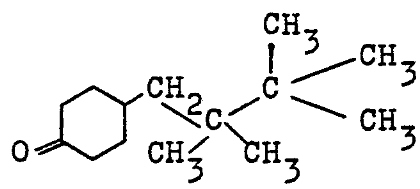

Beispiel 4

Octylcyclohexylester

90 g (0,43 Mol) eines gemäß Beispiel 1 hergestellten Octylcyclohexanols wurden in 200 ml Tetrahydrofuran gelöst und bei 20°C unter Rühren und Eisbadkühlung mit 340 ml einer 15 %igen n-Butyllithiumlösung in Hexan (0,55 Mol) versetzt. Nach einer Stunde wurden unterhalb 30°C 86 g (0,6 Mol) Methyljodid zugetropft. Man ließ 6 Stunden bei 20°C rühren und hydrolysierte anschließend durch Zugabe von 200 ml Wasser. Die organische Phase wurde abgetrennt, mit Wasser neutral gewaschen und bei 50°C und 20 bar die Lösungsmittel abdestilliert. Das Reaktionsprodukt wurde fraktioniert destilliert, wobei 83 g Octylcyclohexylmethyläther erhalten wurden (Ausbeute: 86 %).
Kp 98 - 101°C/0,005 bar; $n_D^{25}$ = 1,4754
Geruch: grün, krautig, rabarberartig

Beispiel 5

Herstellung von 4-Octyl-1-formyl-cyclohexan

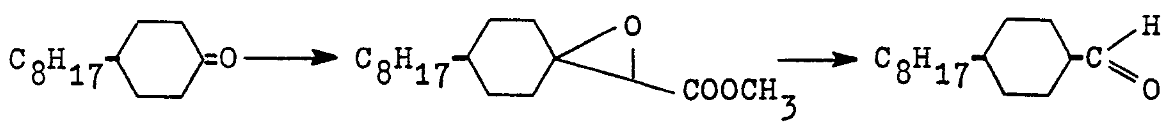

48 g (0,88 Mol) Natriummethylat-Pulver wurden in 250 ml Äther suspendiert und zu dieser Suspension unter Rühren und Kühlung (mit einem Eisbad) eine Lösung von 105 g (0,5 Mol) eines gemäß Beispiel 3 hergestellten Octylcyclohexanons und 85 g (0,78 Mol) Chloressigsäuremethylester so zugetropft, daß die Reaktionstemperatur 40°C nicht überstieg. Nach 16 Stunden Nachreaktion bei Raumtemperatur wurde unter Eiskühlung mit einer Lösung von 30 g Essigsäure in 300 ml Wasser hydrolysiert. Die organische Phase wurde abgetrennt, mit Wasser neutral gewaschen und das Lösungsmittel bei 50°C und 20 bar abdestilliert. Das Reaktionsprodukt wurde fraktioniert, wobei 115 g des Glycidesters erhalten wurden (Ausbeute: 81 %). Kp 140 - 146°C/0,01 bar; $n_D^{25}$ = 1,4760.

50 g (0,18 Mol) des erhaltenen Glycidesters wurden mit 10 g (0,25 Mol) Natrimhydroxid in 100 ml Wasser bei 50°C verseift, die Lösung anschließend mit 2 n Schwefelsäure bis pH 2 angesäuert, 50 ml Tetrahydrofuran zugesetzt und das Reaktionsgemisch so lange auf 60°C erwärmt, bis keine Kohlendioxidabspaltung mehr zu beobachten war. Anschließend wurde mit 500 ml Wasser verdünnt, mit 10 %iger Natronlauge neutralisiert und mit Äther extrahiert. Nach Abdestillieren des Äthers wurde das Reaktionsprodukt fraktioniert. Man erhielt 18 g 4-Octyl-1-formyl-cyclohexan (Ausbeute: 44 %). Kp 78 - 81°C/0,01 bar; $n_D^{25}$ = 1,4731

Geruch: grün, aldehydig, kürbis-melonenartig.

00005196



## Patentansprüche

1. Octylcyclohexanderivate der allgemeinen Formel I

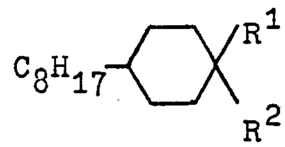

(I),

in der $R^1$ für H und $R^2$ für -OH; -CHO; $-O-CO-CH_3$; $-O-CO-C_2H_5$, $-O-CO-C_3H_7$ oder $-O-CH_3$ steht oder aber $R^1$ und $R^2$ zusammen für =O stehen und der Octylrest $C_8H_{17}$ im wesentlichen als 2,2,3,3-Tetramethylbutylrest vorliegt.

2. Verfahren zur Herstellung des Octylcyclohexanderivates der allgemeinen Formel I gemäß Anspruch 1, in der $R^1$ für H und $R^2$ für -OH steht, dadurch gekennzeichnet, daß man das entsprechende Octylphenol in an sich bekannter Weise katalytisch hydriert.

3. Verfahren zur Herstellung des Octylcyclohexanderivates der allgemeinen Formel I gemäß Anspruch 1, in der $R^1$ für H und $R^2$ für $-O-CO-CH_3$, $-O-CO-C_2H_5$ oder $-O-CO-C_3H_7$ steht, dadurch gekennzeichnet, daß man
   A das entsprechende Octylphenol in an sich bekannter Weise katalytisch hydriert und
   B das erhaltene Octylcyclohexanol in an sich bekannter Weise verestert.

4. Verfahren zur Herstellung des Octylcyclohexanderivates der allgemeinen Formel I gemäß Anspruch 1, in der $R^1$ für H und $R^2$ für $-O-CH_3$ steht, dadurch gekennzeichnet, daß man

A das entsprechende Octylphenol in an sich bekannter Weise katalytisch hydriert und

C das erhaltene Octylcyclohexanol in an sich bekannter Weise methyliert.

5. Verfahren zur Herstellung des Octylcyclohexanderivates der allgemeinen Formel I gemäß Anspruch 1, in der $R^1$ und $R^2$ zusammen für =O stehen, dadurch gekennzeichnet, daß man

A das entsprechende Octylphenol in an sich bekannter Weise katalytisch hydriert und

B das erhaltene Octylcyclohexanol in an sich bekannter Weise mit Chromschwefelsäure, Mangandioxid oder Nickelperoxid oxydiert oder aber katalytisch dehydriert.

6. Verfahren zur Herstellung des Octylcyclohexanderivates der allgemeinen Formel I gemäß Anspruch 1, in der $R^1$ für H und $R^2$ für $-C\overset{O}{\underset{H}{<}}$ steht, dadurch gekennzeichnet, daß man

A das entsprechende Octylphenol in an sich bekannter Weise katalytisch hydriert,

D das erhaltene Octylcyclohexanol in an sich bekannter Weise zum Octylcyclohexanon dehydriert,

E aus dem Octylcyclohexanon durch Umsetzen mit Halogenessigestern in an sich bekannter Weise den Glycidester herstellt und

F diesen durch alkalische Hydrolyse und Säurespaltung zum Aldehyd abbaut.

7. Kosmetische Zubereitungen, enthaltend die Octylcyclohexanderivate der allgemeinen Formel I gemäß Anspruch 1 als Duftstoffe.

8. Wasch- und Reinigungsmittel, enthaltend die Octylcyclohexanderivate der allgemeinen Formel I gemäß Anspruch 1 als Duftstoffe.

9. Leime oder Anstrichmittel, enthaltend die Octylcyclohexanderivate der allgemeinen Formel I gemäß Anspruch 1 als Duftverbesserer.

00005196

Europäisches Patentamt

# EUROPÄISCHER RECHERCHENBERICHT

Nummer der Anmeldung

EP 79 101 121.6

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | Chemical Abstracts, Band 62, Nr. 11, 1965 (COLUMBUS, OHIO, USA) L.A. KHEIFITS et al. "Perfumes from alkylphenols. VI. Synthesis of perfumes from octylphenols" Spalte 12 971 d-e und 12 972 a & Zh. Organ. Khim., Band 1, Nr. 1, 1965 Seiten 89 bis 92 -- | 2 |
| A | GB - A - 1 025 438 (GENERAL ANILINE & FILM CORP.) * Beispiel 6 * -- | 1,2,5 |
| A | Chemical Abstracts, Band 31, Nr. 10, 1937 (COLUMBUS, OHIO, USA) J.B. NIEDERL et al. "Synthesis of long-chain substituted isocyclics and similarly substituted adipic acids. The preparation of γ-t-octylcyclohexanol hexene, hexanone, hydroxylamine, amine, phenol and ß-t-octyladipic acid" Spalten 3885, 8-9 und 3886-1 & Journal of the American Chemical Society Band 59, 1937, Seiten 715 bis 717 -- ./.. | 2 |

KLASSIFIKATION DER ANMELDUNG (Int.Cl.²)

C 07 C 35/08
A 61 K 7/46
C 07 C 47/32
C 07 C 43/18
C 07 C 49/30
C 07 C 69/14
C 07 C 69/24
C 11 D 3/16
C 11 D 3/20

RECHERCHIERTE SACHGEBIETE (Int. Cl.²)

A 61 K 7/46
C 07 C 35/08
C 07 C 43/18
C 07 C 47/32
C 07 C 49/30
C 07 C 69/00
C 11 D 3/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 17-07-1979 | KNAACK |

00005196

Europäisches Patentamt

# EUROPÄISCHER RECHERCHENBERICHT

Nummer der Anmeldung

EP 79 101 121.6
- Seite 2 -

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| A | CHEMISCHES ZENTRALBLATT, Band 107, 1936, 1. Halbjahr, Berlin<br>H.A. BRUSON et al. "Hydroaromatische Alkohole"<br>Seite 3909<br>& US - A - 2 026 668 (RÖHM & HAAS)<br>-- | 2 | |
| A | Chemical Abstracts, Band 31, Nr. 10,1937 (COLUMBUS, OHIO, USA)<br>J.B. NIEDERL et al. "Synthesis of long-chain substituted isocyclics and similarly substituted adipic acids. The preparation of α-t-octylcyclohexanone and a method of indirect structure proof for o- and p-alkylphenols"<br>Spalte 3886-2<br>& Journal of the American Chemical Society Band 59, 1937, Seiten 717 bis 718<br>-- | 5 | RECHERCHIERTE SACHGEBIETE (Int. Cl.²) |
| A | US - A - 2 121 472 (J.B. NIEDERL et al.)<br>* Beispiel 2 *<br>---- | 5 | |